# EUROPEAN PATENT APPLICATION

(11) **EP 3 005 951 A1**
(43) Date of publication of application: **13.04.2016**
(21) Application number: 15001907.3
(22) Date of filing: 26.06.2015
(51) Int. Cl.: A61B 10/06, A61B 10/04

(54) **CATHETER FOR OPTICAL BIOPSY**

(30) Priority: 10.10.2014 CZ 20140691
(71) Applicant: Oprox, a.s., 63900 Brno (CZ)
(72) Inventor: Votruba, Jirí, CZ-1500 Praha 5 (CZ); Bruha, Tomás, CZ-15800 Praha 5 (CZ); Cech, Vladimír, CZ-62300 Brno (CZ); Vítek, Jirí, CZ-58605 Jihlava (CZ)
(74) Representative: Markes, Libor

(57) **Abstract**

A catheter for optical biopsy comprises a forceps (2) for tissue sampling on its distal end and a forceps control device (1), a light source and a device for imaging and analysis of a scanned tissue on its proximal end, with the distal and the proximal ends interconnected with an elongated sheath (4) containing a Bowden (3) with a control cable or wire and at least one optical fibre (5). The sheath (4), made of flexible plastic, provides a lengthwise channel (6) for the Bowden (3) placement, whereas the channel (6) is open with a fissure (7) along the full length of the sheath (4).

## Description

### Technical Field

The present invention relates to a catheter specifically designed for pulmonary tissue biopsy comprising optical fibre allowing for the tissue imaging before sampling. The catheter further includes forceps on the distal end for tissue sample taking and forceps controls, a light source and a device for evaluation and analysis of the scanned tissue on the proximal end. The distal and the proximal end are interconnected with an oblong sheath with a Bowden control cable or wire and at least one optical fibre.

### Background Art

Biopsy forceps with optical fibre are described in many patent documents. For example US 5762613 describes an integrated device combining biopsy forceps and optical fibre. The device comprises an elongated catheter for insertion into the human body and navigation to the area of interest. Optical fibre passing through the device is connected to an electro-optical device for spectral analysis on the proximal end, i.e. on the examiner side, and illuminates and scans the tissue on the distal end. The distal end is further equipped with a pair of jaws controlled with a handle on the proximal end of the catheter via a pair of control wires passing through the catheter. The catheter body comprises a flexible metal spiral wound tube with optical fibre in a plastic insulator passing through its centre. The pair of control wires is placed between the insulator and the spiral tube.

US 2009326384 describes a device used for biopsy and comprising an integrated probe of several optical fibres. While light energy is supplied to the tissue by one of the fibres, another fibre scanning the image for spectrometer evaluation. The device is further equipped with biopsy forceps on the distal end and forceps controls on the proximal end. The catheter is placed in an elongated Teflon sheath where in one embodiment of the invention there is a flexible metal central tube filled with plastic holding a pair of optical fibres and a forceps control wire in the centre between them. In another embodiment of the invention the sheath consists of two closed mutually separated channels, the bigger one holding a control wire and the smaller one optical fibre.

A catheter to be used for pulmonary tissue sampling should meet specific requirements: Its diameter should not exceed 2.8 mm and its material should be very flexible. Regarding the structure of the catheters described in the abovementioned patent documents they clearly cannot meet these requirements.

Instruments made of surgical steel are assumed to be sterilised by autoclave heat. Instruments combining surgical steel and certain plastic materials cannot be sterilised in this way for plastics do not resist high temperatures. This in any case applies to organic optical fibre the use of which in catheters is dictated by the need to preserve the required flexibility. Therefore single use of these instruments and therefore their disposal after every intervention are assumed. And yet the production costs of a catheter, especially its metal part, are high and hence use of such catheters burdens the economy of the healthcare facilities using them.

The purpose of the present invention is to design a catheter for optical biopsy, especially of pulmonary tissues, considerably reducing costs of the intervention.

### Disclosure of the Invention

This task is resolved by a catheter for optical biopsy with forceps on the distal end for tissue sample taking and forceps controls, a light source and a device for evaluation and analysis of the scanned tissue on the proximal end. The distal and the proximal ends are interconnected with an oblong sheath containing a Bowden with a control cable or wire and at least one optical fibre. The sheath made of flexible plastic is provided with a lengthwise channel for Bowden placement. The channel is open with a fissure passing through the full length of the sheath.

The wall of the channel for the Bowden placement may provide at least one groove for the optical fibre placement.

At least one end of the sheath may be provided with a grip collar for the sheath reinforcement.

In a preferred embodiment of the catheter two organic optical fibres are placed in the sheath with their distal ends mutually axially displaced by 2.5 to 4.5 mm for optimum imaging achievement.

The sheath may hold at least three organic optical fibres, one for illumination and the other two or more for detection, whereas the distal ends of the detection fibres are axially displaced in relation to the end of the illumination fibre by distance 1 to 10 mm.

### Brief Description of the Drawing

Embodiments of the present invention are described with reference to the accompanying drawing:
- Fig. 1: shows axonometric projection of the proximal end of the catheter, while Figs. 2 to 4, in a bigger scale, show the distal end of the catheter,
- Fig. 2: in axonometric projection,
- Fig. 3: in side projection and
- Fig. 4: in horizontal projection,
- Fig. 5: shows a cross-section of a sheath with a Bowden and two optical fibres and
- Fig. 6: shows a cross-section of an empty sheath.

### Preferred Embodiments of the Invention

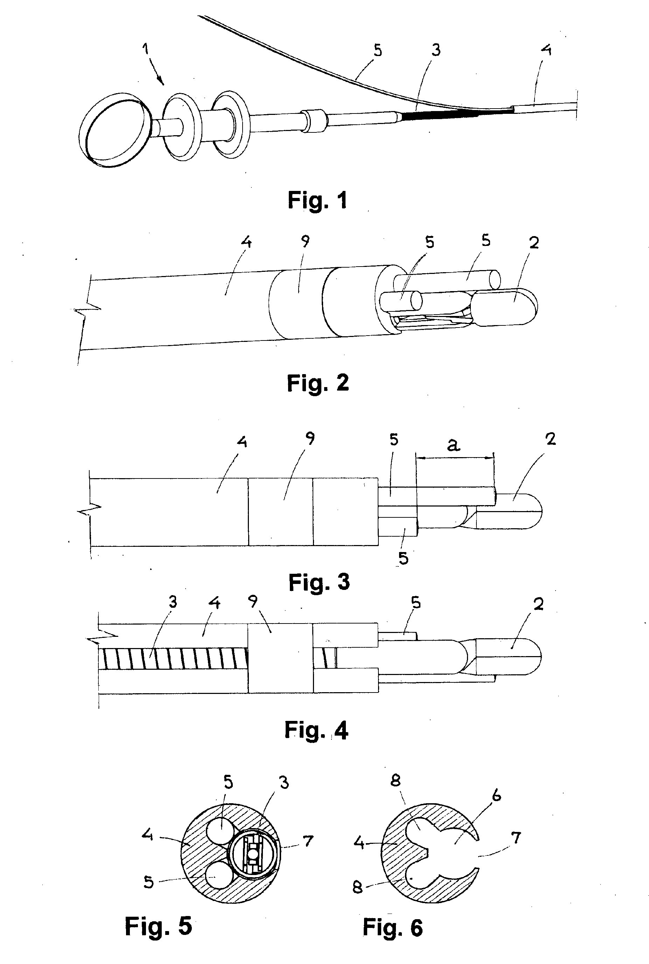

A catheter for optical biopsy shown in Figs. 1 to 6 combines biopsy forceps and an optical device for tissue imaging and analysis before sampling. On the proximal end, shown in Fig. 1, there is a known forceps control device **1** connected with the forceps **2** by a control cable passing through a Bowden **3.** The Bowden **3** is placed in a sheath **4,** together with a pair of organic optical fibres **5** on the proximal end of the device stitching out of the sheath **4** and connected to a light source and a device for spectral analysis, not shown in the drawing. On the distal end of the catheter, or Bowden **3,** see Figs. 2 to 4, there is a pair of forceps **2** for tissue sampling. The sheath **4** is made of flexible plastic and comprises a lengthwise channel **6** for Bowden **3** laying. The channel **6** is open with a fissure **7** along the full length of the sheath **4.** The wall of the channel **6** for the Bowden **3** laying provides two grooves **8** for two optical fibres **5,** one of the fibres **5** for the tissue illumination and the other fibre **5** for back transmission of the image for spectral analysis. Locations of the channel **6** and the grooves **8** in the sheath **4** are apparent from Figs. 5 and 6, showing sheath **4** cross-sections. For the purpose of reinforcement of the distal end the sheath **4** is provided with a metal grip collar **9.** The distal ends of the optical fibres **5** stitching out of the sheath **4** are mutually axially displaced by distance **a** = 3.5 mm.

The catheter construction pursuant to the present invention allows for its assembly *in situ* before the intervention and separate handling of the metal structure of the biopsy forceps **2** and the plastic sheath assembly **4** with the organic optical fibres after the intervention. Before the intervention the suture nurse will take the biopsy forceps out of the sterilizer and the sheath **4** with the optical fibre **5** assembly sterilised by the manufacturer from the sterile package. In a sterile environment and using a special instrument the suture nurse will press the Bowden **3** with the cable to the sheath **4** channel **6** through the fissure **7** across the full length of the sheath **4.**

To reinforce the sheath **4** ends the suture nurse will slide the collars **9** on them and grip with a forceps. Now the device will be ready for use. After the intervention is complete and the tissue is taken out of the forceps **2** the grip collars **9** will be cut, the Bowden **3** will be taken out of the sheath **4,** and the sheath **4** which the optical fibres **5,** which cannot be treated with high temperatures, will be disposed of, while the metal biopsy forceps **2,** whose price is an order higher than the price of the plastic part, will be ready for further use after cleaning and autoclaving.

## Claims

1. A catheter for optical biopsy with a forceps (2) for tissue sampling on its distal end and a forceps control device (1), a light source and a device for imaging and analysis of a scanned tissue on its proximal end, with the distal and the proximal ends interconnected with an elongated sheath (4) containing a Bowden (3) with a control cable or wire and at least one optical fibre (5), **characterised in that** the sheath (4), made of flexible plastic, provides a lengthwise channel (6) for the Bowden (3) placement, whereas the channel (6) is open with a fissure (7) along the full length of the sheath (4).

2. The catheter of claim 1, **characterised in that** at least one groove (8) for optical fibre (5) laying is made in the wall of the channel (6) for the Bowden (3) placement.

3. The catheter of clams 1 or 2, **characterised in that** at least one of its ends is reinforced with a grip collar (9).

4. The catheter of claims 1 to 3, **characterised in that** the sheath (4) holds two organic optical fibres (5) with their distal ends mutually axially displaced by distance (a) 2.5 to 4.5 mm.

5. The catheter of claims 1 to 3, **characterised in that** the sheath (4) contains at least three organic optical fibres (5), one for illumination and the other ones for detection, whereas the distal ends of the detection fibres are axially displaced in relation to the illumination fibre by distance 1 to 10 mm.
